(19) Europäisches Patentamt
European Patent Office
Office européen des brevets

(11) **EP 4 389 133 A1**

(12) **EUROPEAN PATENT APPLICATION**
published in accordance with Art. 153(4) EPC

(43) Date of publication:
**26.06.2024 Bulletin 2024/26**

(21) Application number: **22858399.3**

(22) Date of filing: **09.08.2022**

(51) International Patent Classification (IPC):
*A61K 33/08* (2006.01)          *A61K 9/20* (2006.01)
*A61K 47/32* (2006.01)          *A61K 47/36* (2006.01)
*A61K 47/38* (2006.01)          *A61P 1/04* (2006.01)
*A61P 1/10* (2006.01)          *A61P 3/02* (2006.01)

(52) Cooperative Patent Classification (CPC):
**A61K 9/20; A61K 33/08; A61K 47/32; A61K 47/36;
A61K 47/38; A61P 1/04; A61P 1/10; A61P 3/02**

(86) International application number:
**PCT/JP2022/030484**

(87) International publication number:
**WO 2023/022078 (23.02.2023 Gazette 2023/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.08.2021  JP 2021134883**

(71) Applicant: **SETOLAS Holdings, Inc.
Takamatsu-shi, Kagawa 760-0026 (JP)**

(72) Inventor: **YAMAJI, Toru
Kita-gun, Kagawa 761-0705 (JP)**

(74) Representative: **Vossius & Partner
Patentanwälte Rechtsanwälte mbB
Siebertstrasse 3
81675 München (DE)**

(54) **MAGNESIUM OXIDE TABLETS**

(57)    The purpose of the present invention is to provide tablets which contain magnesium oxide particles capable of achieving both of the reduction in friability and the reduction in a capping occurrence rate as a main component. Provided are tablets each of which comprises granules comprising both of magnesium oxide particles and an internal additive and an external additive, in which at least cellulose and/or a cellulose derivative is contained as the internal additive and the external additive and the [cellulose and/or a cellulose derivative contained as the internal additive]:[cellulose and/or a cellulose derivative contained as the external additive] ratio falls within the range from 75:25 to 10:90 by mass.

EP 4 389 133 A1

**Description**

FIELD

The present invention relates to a tablet containing magnesium oxide particles as a main ingredient.

BACKGROUND

Tablets containing magnesium oxide particles as a main ingredient are known and widely used as tablets for various applications such as antacid, laxative, magnesium supplementation, and anti-hypomagnesemia. However, magnesium oxide tablets have poor formability and therefore exhibits high friability, resulting in problems such as cracking and capping during transportation and shipping. Occurrence of abrasion and capping results in the problem of causing cracking in tablets during production, transportation, and dispensing. To obtain tablets with high hardness, low friability, and good formability, it is a common practice to compress a mixture powder with low bulk density. On the other hand, however, capping tends to occur when air is trapped inside the tablet during compression. Therefore, the challenge is to reduce both the friability and the incidence of capping.

Various attempts have been made to reduce capping. For example, Patent Literature 1 discloses an example in which capping caused by abrasion between the die and the punch is addressed by blending a binder and a disintegrant during dry granulation to thereby reduce the abrasion between the die and the punch during tableting. Patent Literature 2 describes that capping caused by air not escaping during compression can be suppressed by designing the shape of the punch so as to allow air to escape easily.

For magnesium oxide tablets, cellulose or cellulose derivatives are sometimes added as highly compressible and plastic deformable additives. However, due to concerns about reduced flowability, delayed disintegration time caused by agglomeration of magnesium oxide particles, reduced tube passage, and increased incidence of capping, it is technical common knowledge that these additives may be added only before granulation but not after granulation. Nevertheless, Patent Literature 2 attempts to improve the long-term sustainability and strength of short-time disintegration by using two types of disintegrants and two types of binders (crystalline cellulose) in the formulation, and adding them both before and after granulation. However, the improvement of the capping rate has been achieved only by improving the shape of the tablets.

CITATION LIST

Patent Literature

    [Patent Literature 1] JP2003-146889 A
    [Patent Literature 2] JPWO2011/030659 A

SUMMARY

Problem to be Solved

A problem to be solved by the present invention is to provide magnesium oxide tablets that can reduce both the friability and the incidence of capping.

Means to solve the Problem

The present inventors have made diligent studies to reduce both the friability and the incidence of capping in tablets containing magnesium oxide particles as the main ingredient. As a result, the present inventors have found that tablets with both reduced friability and reduced incidence of capping can be obtained by optimizing the ratios of a cellulose and/or a cellulose derivative to be used as an internal additive and as an external additive, and have also found their appropriate ratios. Based on these findings, the present inventors have completed the present invention.

One or more embodiments of the present invention provide the following.

    [1] A tablet comprising magnesium oxide as an active ingredient, wherein the tablet comprising:

        granules containing magnesium oxide particles and an internal additive; and

an external additive,
wherein each of the internal additive and the external additive at least contains a cellulose and/or a cellulose derivative,
wherein the mass ratio between [the cellulose and/or the cellulose derivative contained as an internal additive] : [the cellulose and/or the cellulose derivative contained as an external additive] is within the range of from 75:25 to 10:90.

[2] The tablet according to [1], wherein the cellulose and/or the cellulose derivative is at least one selected from the group consisting of crystalline cellulose, microcrystalline cellulose, powder cellulose, methyl cellulose, ethyl cellulose, carboxyl methyl cellulose, carboxyl methyl cellulose calcium, carboxyl methyl cellulose sodium, hydroxy ethyl cellulose, hydroxy propyl cellulose, hydroxy propyl methyl cellulose, and low substituted hydroxy propyl cellulose.
[3] The tablet according to [1] or [2], wherein the content ratio of magnesium oxide particles in the total tablet is 80 to 90 mass %.
[4] The tablet according to any one of [1] to [3], wherein the granules contain at least one selected from the group consisting of croscarmellose sodium, corn starch, carmellose calcium, crospovidone and carboxyl starch sodium at an amount of 1 to 3.5 mass % of the total tablet.
[5] A method for producing a tablet containing magnesium oxide as an active ingredient tablet, comprising:

mixing magnesium oxide particles with an internal additive including a cellulose and/or a cellulose derivative to prepare a mixture;
granulating the mixture to prepare granules; and
adding an external additive including a cellulose and/or a cellulose derivative to the granules and tableting the granules;
wherein the mass ratio between [the cellulose and/or the cellulose derivative contained as an internal additive] : [the cellulose and/or the cellulose derivative contained as an external additive] is within the range of from 75:25 to 10:90.

[6] The method according to [5], wherein the cellulose and/or the cellulose derivative is at least one selected from the group consisting of crystalline cellulose, microcrystalline cellulose, powder cellulose, methyl cellulose, ethyl cellulose, carboxyl methyl cellulose, carboxyl methyl cellulose calcium, carboxyl methyl cellulose sodium, hydroxy ethyl cellulose, hydroxy propyl cellulose, hydroxy propyl methyl cellulose and low substituted hydroxy propyl cellulose.
[7] A tablet produced by a method according to [5] or [6].

Effects of the Invention

According to the present invention, both the friability and the incidence of capping in tablets containing magnesium oxide particles as a main ingredient can be reduced, by adjusting the ratios of a cellulose and/or a cellulose derivative to be used as an internal additive and as an external additive. Reducing both the friability and the incidence of capping makes it possible to reduce the occurrence of cracking during manufacturing, transportation, and dispensing.

EMBODIMENTS

The present invention is described hereinafter in detail with reference to specific embodiments thereof. However, the present invention is not limited to the following embodiments and can be carried out in any embodiment that does not deviate from the gist according to the present invention.

[Overview]

As mentioned above, magnesium oxide tablets have poor formability, resulting in high friability and causing the problem of capping. However, if the hardness is increased in order to reduce the formability, the incidence of capping increases as the formability improves, due to air entrapment during compression. Therefore, the challenge is to reduce both the friability and the incidence of capping. In this regard, Patent Literature 2 reports various studies conducted using two types of disintegrants and two types of binders (crystalline cellulose) and adding them both before and after granulation. However, Patent Literature 2 did not come up with the possibility of reducing both the friability and the incidence of capping by adjusting the ratios of a cellulose and/or a cellulose derivative to be used as an internal additive and as an external additive.
The present inventors have made diligent studies to reduce both the friability and the incidence of capping, and have surprisingly found that tablets with both reduced friability and reduced incidence of capping can be obtained by adding a cellulose and/or a cellulose derivative both before and after granulation, and by optimizing the ratios of the cellulose and/or the cellulose derivative to be added before granulation as an internal additive and that to be added after granulation as an external additive. The present invention has been made based on such surprising findings.

[Friability]

The term "friability" as used herein refers to an index indicating the degree of abrasion or brittleness of tablets against impact, and can be measured in accordance with the method described in Japanese Pharmacopoeia, 17th Edition, General Information, Tablet Friability Test." Specifically, tablets with an amount close to 6.5 g are subjected to a abrasion test of 100 rotations (at 24 to 26 rpm) using a tablet abrasion tester (TFT-1200, manufactured by Toyama Sangyo Co., Ltd.), and the initial mass of the tablets before abrasion and the mass of the tablets after abrasion are measured. The friability is calculated according to Formula 1 below.
[Formula 1]

$$\text{Friability} = \frac{\{(\text{Initial mass of tablets (g)}) - (\text{Mass of tablets after abrasion (g)})\}}{(\text{Initial mass of tablets (g)})} \times 100 \qquad \text{Formula 1}$$

When the friability of the tablet of the present invention is measured by the method mentioned above, the upper limit of the friability may preferably be less than 0.40%, more preferably less than 0.35%, or less than 0.30%. On the other hand, the lower limit of the friability is not specified because the lower the friability, the better.

[Capping]

The term "capping" as used herein refers to the phenomenon that the top or bottom portion of a tablet peels off as a sectional piece. This can occur by impacts during tableting, transportation, and dispensing in the tablet production process. The incidence of capping can be determined by a cassette rotor test. Specifically, tablets are ejected from a cassette rotor (TOSHO, for Magmit tablets 500 mg) set at a height of 2 m, and the number of tablets that have caused capping is counted. After a sufficient number of tablets (e.g., 100 tablets) have been tested, the percentage of tablets that have caused capping to the total number of tablets tested can be calculated according to Formula 2 below. Examples of the flooring material to be used in this test include Chemicrete E (ABC Trading Co., Ltd.), which is a combination of concrete metal trowel + epoxy-coated flooring material + paste method (thickness: 2 mm), or its equivalent materials.
[Formula 2]

$$\text{Incidence of capping (\%)} = \frac{(\text{Number of tablets that have caused capping})}{(\text{Total number of tablets tested})} \times 100 \qquad \text{Formula 2}$$

When the incidence of capping of the tablet of the present invention is measured by the method mentioned above, the upper limit of the incidence of capping may preferably be less than 12%, more preferably less than 11%, or less than 10%. On the other hand, the lower limit of the incidence of capping is not specified because the lower the incidence of capping, the better.

[Hardness]

The term "hardness" as used herein refers to an index indicating the hardness of a tablet, and can be measured with a commercially available tablet hardness tester. Specifically, the hardness in the diameter direction of a tablet can be measured using, e.g., a tablet hardness tester such as DC-50 (Okada Seiko Co., Ltd.). From the viewpoint of achieving the effect of the present invention, if the hardness is too low, the friability increases. Accordingly, the lower limit of the hardness may preferably be 30 N or more, more preferably 40 N or more, or 50 N or more. On the other hand, the upper limit of the hardness is not specified because the higher the hardness, the better.

[Disintegration time]

The term "disintegration time" as used herein refers to an index indicating a tablet's tendency to disintegrate in a solution. The disintegration time of a tablet can be measured according to the method described in Japanese Pharmacopoeia, 17th Edition, General Information, Disintegration Test. Specifically, the disintegration time can be determined using a disintegration tester (NT-20HS, Toyama Sangyo Co., Ltd.), by measuring the disintegration time of an appropriate number of test tablets (e.g., 6 tablets) in a test medium (e.g., water). An appropriate disintegration time is desirable because it provides easy-to-swallow tablets that disintegrate in a short time in the oral cavity when taken. The upper limit of the appropriate disintegration time may preferably be 20 seconds or less, more preferably 15 seconds or less, or 11 seconds or less. The lower limit is not particularly restricted, but may be usually 5 seconds or more, or 1 second or more.

[Cellulose and/or cellulose derivative]

The term "cellulose" as used herein refers to a linear polymer represented by $(C_6H_{10}O_5)_n$, in which $\beta$-glucose molecules are polymerized into a linear chain via glycosidic bonds. Examples of cellulose include crystalline cellulose, microcrystalline cellulose, and powder cellulose. The term "cellulose derivative" as used herein refers to a molecule derived from a cellulose molecule by introducing a different substituent into a hydroxy group thereof via an ether or ester bond. Examples of cellulose derivatives include methyl cellulose, ethyl cellulose, carboxyl methyl cellulose, carboxyl methyl cellulose calcium, carboxyl methyl cellulose sodium, hydroxy ethyl cellulose, hydroxy propyl cellulose, hydroxy propyl methyl cellulose, and low substituted hydroxy propyl cellulose. The term "cellulose and/or cellulose derivative" as used herein refers to at least one substance selected from the "celluloses" and "cellulose derivatives" defined above. The term "low substituted hydroxy propyl cellulose" as used herein refers to a cellulose with a very small amount of hydroxypropoxy groups introduced into its glucose rings, i.e., cellulose having a low level of O-(2-hydroxypropyl) substitution with a molar substitution degree = 0.2 to 0.4. Examples of shapes of such cellulose derivatives include powder, particles, and microparticles.

Celluloses and/or cellulose derivatives may be synthesized or commercially available. Commercially available products include crystalline celluloses such as various grades of Ceolus™ from Asahi Kasei Corporation, e.g., Ceolus™ PH-101, UF-711, PH-102, PH-200, PH-301, PH-302, PH-20JP, UF-702, KG-802, and KG-1000.

[Magnesium oxide particles]

Magnesium oxide particles to be used in the present invention are particles of magnesium oxide (MgO). Magnesium oxide particles to be used in the present invention can be obtained by calcining magnesium hydroxide particles. For example, it can be obtained by preparing magnesium hydroxide with an average particle size of 1 to 10 $\mu$m according to laser diffraction scattering and calcining it at 600 to 1000°C. Magnesium oxide particles may be either a synthesized product or a commercially available product. Examples of magnesium oxide particles to be used include: heavy grade from Kyowa Chemical Industry Co., Ltd.; powder grade from Konoshima Chemical Co., Ltd.; light grade from Tomita Pharmaceutical Co., Ltd., and heavy grade of Japanese Pharmacopoeia magnesium oxide.

Magnesium oxide particles to be used in the present invention may be either in powder form or in granular form, but granular form is more effective in preventing abrasion by the tableting machine, and also produces tablets with higher contents and better shape retention stability.

Although not limited, magnesium oxide particles to be used in the present invention may preferably have a predetermined particle diameter in some cases. For example, as measured by laser diffraction scattering, the upper limit of the average particle diameter of the magnesium oxide particles may be typically 40 $\mu$m or less, or 20 $\mu$m or less, or 10 $\mu$m or less. When the average particle size of magnesium oxide particles is adjusted to the aforementioned upper limit or less, the resulting tablets may have a smaller suspended particle size upon disintegration and be less coarse in the oral cavity. On the other hand, the lower limit of the average particle diameter is not limited, but may be usually 0.25 $\mu$m or more, or 0.5 $\mu$m or more, or 1 $\mu$m or more, due to manufacturing limitations and cost effectiveness.

The average particle size and average particle diameter of the magnesium oxide particles of the present invention and magnesium hydroxide particles used as raw materials therefor can be measured according to laser diffraction scattering method with a commonly used instrument, such as a particle size analyzer (MT3300EX2 from Microtrac Bell Corporation).

The bulk density of magnesium oxide particles to be used in the present invention may be set to an upper limit of 0.8 g/mL or less, or 0.7 g/mL or less, since too high a density may cause a decrease in hardness. On the other hand, since too low bulk density may cause capping and lamination, the lower limit may be 0.1 g/mL or more, or 0.2 g/mL or more. Bulk density can be measured, e.g., using a 100 mL stainless steel cup (actual mass value (g)/100 (mL)).

The angle of repose of magnesium oxide particles to be used in the present invention may be set to an upper limit of 50° or less, or 48° or less, since an excessively large angle of repose may lead to a variation in the mass of the resulting tablets during tableting due to deteriorated flowability. On the other hand, the lower limit of the angle of repose is not specified because the lower the angle of repose, the better. The angle of repose can be measured, e.g., using Multitester MT-1 from Seishin Enterprise Co., Ltd.

[Tablet]

An aspect of the present invention relates to a tablet containing magnesium oxide as an active ingredient tablet, comprising: granules containing magnesium oxide particles and an internal additive; and an external additive, wherein each of the internal additive and the external additive at least contains a cellulose and/or a cellulose derivative, and wherein the mass ratio between [the cellulose and/or the cellulose derivative contained as an internal additive] : [the cellulose and/or the cellulose derivative contained as an external additive] is within a predetermined range.

Magnesium oxide particles may preferably be contained in the granules together with the internal additive from the viewpoint of achieving the effects of the present invention. The content of magnesium oxide particles may be 90 mass % or less, or 88 mass % or less of the total tablet, since too high a content may result in insufficient formability. On the other hand, if the content of magnesium oxide particles is too low, tablets with high formability can be obtained because the additives with high compressibility and plastic deformability can be blended instead, but the cost per tablet becomes higher. Therefore, the content of magnesium oxide particles may preferably be 80 mass % or more, or 85 mass % or more, of the total tablet.

The tablet of the present invention is characterized in that the mass ratio between [the cellulose and/or the cellulose derivative contained as an internal additive] : [the cellulose and/or the cellulose derivative contained as an external additive] is within a predetermined range. Increasing the ratio of cellulose and/or cellulose derivative to be contained as an external additive can be expected to increase hardness and reduce friability, but increasing the ratio too much can lead to an increase in capping incidence and a decrease in tube passing properties. Accordingly, when the total mass of the cellulose and/or the cellulose derivative contained in the tablet is 100, the mass ratio of cellulose and/or cellulose derivative contained as an external additive may preferably have an upper limit of 90 or less, for example, 88 or less, 85 or less, 80 or less, or 75 or less, and a lower limit of more than 20, or 21 or more, 22 or more, 23 or more, 24 or more, 25 or more. On the other hand, if the ratio of cellulose and/or cellulose derivative contained as internal additives is too low, compaction may be insufficient, resulting in the generation of a large amount of microparticles, which can easily trap air, causing capping in the resulting tablets when air is not released during subsequent tableting. Therefore, a certain amount of cellulose and/or a cellulose derivative should be blended as an internal additive. Conversely, however, if the ratio of cellulose and/or cellulose derivative contained as internal additives is too high, granules obtained by granulation may have already been compacted, and further compacting during tableting makes it difficult for the granules to be broken, which may cause a decrease in formability, i.e., an increase in the friability. Accordingly, when the total mass of the cellulose and/or the cellulose derivative contained in the tablet is 100, the mass ratio of cellulose and/or cellulose derivative contained as an internal additive may preferably have an upper limit of lower than 80, or 79 or less, 78 or less, 77 or less, 76 or less, or 75 or less, and a lower limit of 10 or more, 12 or more, 15 or more, 20 or more, or 25 or more.

The content of cellulose and/or cellulose derivative may be 20% or less, 15% or less, or 12% or less of the total tablet, as too high a content can increase the cost per tablet. On the other hand, if the content is too low, the effect of the present invention may not be achieved. Accordingly, the content may be 5 mass % or more, or 7 mass % or more, or 9 mass % or more of the total tablet.

In the tablet of the present invention, the cellulose and/or cellulose derivative as defined above may preferably be used as an excipient or binder in both internal and external additives. The cellulose and/or cellulose derivative contained in the internal additive and that contained in the external additive may be either the same or different.

The term "internal additive" as used herein refer to an additive, which may include one or more substances, that is added to and mixed with the active ingredient prior to the granulation step in the manufacture of tablets. In addition to the cellulose and/or cellulose derivative as defined above, another additive may also be added as an internal additive. In particular, in addition to the cellulose and/or cellulose derivatives in the internal and external additives in specific ratios as explained above, it may be preferable to add a disintegrant such as croscarmellose sodium, corn starch, calcium carmellose, crospovidone, or sodium carboxystatina as an internal additive, in order to achieve a preferred disintegration time as described above. The disintegrant may be either a synthesized product or a commercially available product. Examples of commercial products that can be used include Kiccolate™ ND-2HS from Nichirin Chemical Industries, Ltd. The upper limit of the disintegrant content may be 5 mass % or less, or 3.5 mass % or less of the total tablet mass, since otherwise the forming of tablets may be difficult. On the other hand, the lower limit of disintegrant content may be 1 mass % or more, or 2 mass % or more of the total tablet, since too little disintegrant content may cause disintegration difficulties.

The term "external additive" as used herein refer to an additive, which may include one or more substances, that is added to and mixed with the granules after the granulation step in the manufacture of tablets. In addition to the cellulose and/or cellulose derivatives in the internal and external additives in specific ratios as explained above, another additive may also be added as an external additive. In particular, it may be preferable to add a lubricant from the standpoint of achieving the effects of the present invention. Examples of lubricants include stearic acid and its salts (Mg, Ca salts), preferably stearic acid salts, among which magnesium stearate and calcium stearate may be preferred. The lubricant may be either a synthesized product or a commercially available product. The upper limit of the amount of lubricant added may be 2 mass % or less, or 1.5 mass % or less, or 1.0 mass % or less of the total tablet, since too much lubricant may lead to delayed disintegration. On the other hand, the lower limit of the amount of lubricant added may be 0.2 mass % or more, or 0.5 mass % or more, or 0.9 mass % or more of the total tablet, since too little lubricant may adhere to the punch and the die.

The shape of the tablet of the present invention is not particularly limited, but from the viewpoint of achieving the effects of the present invention and improving the ease of taking it as an oral drug, the upper limit of the diameter may be 14 mm or less, or 10 mm or less, and the lower limit may be 5 mm or more, or 6 mm or more. The thickness is likewise not restricted, but the upper limit may be 8 mm or less, or 7 mm or less, and the lower limit may be 3 mm or more, or 4 mm or more. For example, for the purpose of suppressing capping further, a shape such as that shown in Patent Literature 2 may be used.

The mass of the tablet of the present invention is not limited as long as it is within the range for oral dosage forms normally used as tablets with magnesium oxide as the active ingredient. For example, the upper limit may be 1000 mg or less, or 800 mg or less, or 600 mg or less per tablet, and the lower limit can be 10 mg or more, or 50 mg or more, or 100 mg or more per tablet.

The tablets of the present invention are orally administered as pharmaceuticals for humans or animals for the purpose of, e.g., antacid, laxative, or prevention of the formation of calcium oxalate in the urinary tract. They can also be used as supplements for humans or animals for the purpose of, e.g., magnesium supplementation or anti-hypomagnesemia. Its dosage depends on the application, purpose, or medical conditions. For example, when it is used as an antacid, a dosage containing 0.5 to 1.0 g of magnesium oxide per adult per day is usually administered orally in several divided doses. When it is used as a laxative, a dosage containing 2 g of magnesium oxide per adult per day is usually administered orally either in three divided doses before or after meals or once before bedtime. When it is used for preventing the formation of calcium oxalate in the urinary tract, a dosage containing 0.2 to 0.6g of magnesium oxide per adult per day is usually administered orally with large amounts of water. For other uses, it is usually taken within the upper tolerable limit of magnesium. For example, for intake from sources other than regular foods, the dietary reference intakes for magnesium equivalent in the U.S. are 350 mg per day for healthy adults and 5 mg per kg of body weight per day for healthy children (Institute of Medicine (IOM). Food and Nutrition Board. "Dietary Reference Intakes: Calcium, Phosphorus, Magnesium, Vitamin D and Fluoride". Washington, DC: National Academy Press, 1997).

The pharmaceutical product according to the present invention has been approved in Japan, based on its active ingredient, for applications as an antacid, laxative, and agent for preventing the formation of calcium oxalate in the urinary tract. The pharmaceutical product according to the present invention may further contain one or two or more other optional components as internal and/or external additives, as long as they do not substantially interfere with the expected effects in each of these applications or with the effects of reducing friability and capping incidence. Examples of such other ingredients include, but are not limited to, various pharmaceutically acceptable additives, such as dyes and flavor agents. Any one of these ingredients may be used alone, or any two or more of these ingredients may be used together in any combination and any ratios.

[Method for producing the tablets]

Another aspect of the present invention relates to a method for producing a tablet containing magnesium oxide as an active ingredient, comprising:

mixing magnesium oxide particles with an internal additive including a cellulose and/or a cellulose derivative to prepare a mixture;
granulating the mixture to prepare granules; and
adding an external additive including a cellulose and/or a cellulose derivative to the granules and tableting the granules;
wherein the mass ratio between [the cellulose and/or the cellulose derivative contained as an internal additive] : [the cellulose and/or the cellulose derivative contained as an external additive] is within the range of from 75:25 to 10:90.

First, magnesium oxide particles are mixed with an internal additive, which includes a cellulose and/or a cellulose derivative as well as one or more other ingredients optionally used, via a known method.
The resulting mixture is then granulated into granules. In view of the ingredients of the tablet of the present invention, dry granulation may be preferred. For example, granules can be produced by granulating with a commercially available dry granulator (RC-156 dry granulator from Freund Corp.) under normal granulation conditions.

The thus-produced granules are mixed with an external additive, which includes a cellulose and/or a cellulose derivative as well as one or more other ingredients optionally used, and the mixture is then tableted. Any method can be used for the addition of the external additive as long as it allows for the addition of the external additive around the granules. The pressure for punching per tablet may be set with an upper limit of 20 kN or less, or 18 kN or less, or 16 kN or less, and a lower limit of 2 kN or more, or 3 kN or more, or 4 kN or more. The shape of the punch may be a standard R, a two-tiered R, a sugar-coated R, a sharp-cornered R, a sharp-cornered flat plane, a round-cornered flat plane, or any other shape.

Although various aspects of the present invention have been described above, the present invention is not limited to these aspects. As will be clear to a person skilled in the art, it is also possible to extract other optional aspects of the present invention from the detailed description above and the examples described below.

EXAMPLES

The present invention will be described in more detail in the following examples. However, these examples are only shown for convenience of explanation, and the present invention is not limited to these examples in any sense.

[Formulation]

Tablets were manufactured using the formulations shown in the following table.

[Table 1]

| Lot No. | | Comparative Example 1 | Comparative Example 2 | Example 1 | Example 2 | Example 3 | Comparative Example 3 |
|---|---|---|---|---|---|---|---|
| [Internal additive cellulose] : [External additive cellulose] | | 100:0 | 80:20 | 75:25 | 50:50 | 25:75 | 0:100 |
| Granules (active ingredient + internal additive) | Magnesium oxide | 520.0 mg | 520.0 mg | 520.0 mg | 520.0 mg | 520.0 mg | 520.0 mg |
| | Crystalline cellulose | 60.0 mg | 48.0 mg | 45.0 mg | 30.0 mg | 15.0 mg | 0 mg |
| | Croscarmellose sodium | 16.0 mg | 16.0 mg | 16.0 mg | 16.0 mg | 16.0 mg | 16.0 mg |
| External additive | Crystalline cellulose | 0 mg | 12.0 mg | 15.0 mg | 30.0 mg | 45.0 mg | 60.0 mg |
| | Calcium stearate | 6.0 mg | 6.0 mg | 6.0 mg | 6.0 mg | 6.0 mg | 6.0 mg |
| Total | | 602.0 mg | 602.0 mg | 602.0 mg | 602.0 mg | 602.0 mg | 602.0 mg |

[Table 2]

| Lot No. | | Comparative Example 4 | Comparative Example 5 | Example 4 | Example 5 | Example 6 | Comparative Example 6 |
|---|---|---|---|---|---|---|---|
| [Internal additive cellulose] : [External additive cellulose] | | 100:0 | 80:20 | 75:25 | 50:50 | 25:75 | 0:100 |
| Granules (active ingredient + internal additive) | Magnesium oxide | 520.0 mg | 520.0 mg | 520.0 mg | 520.0 mg | 520.0 mg | 520.0 mg |
| | Crystalline cellulose | 60.0 mg | 48.0 mg | 45.0 mg | 30.0 mg | 15.0 mg | 0 mg |
| | Croscarmellose sodium | 16.0 mg | 16.0 mg | 16.0 mg | 16.0 mg | 16.0 mg | 16.0 mg |
| External additive | Crystalline cellulose | 0 mg | 12.0 mg | 15.0 mg | 30.0 mg | 45.0 mg | 60.0 mg |
| | Calcium stearate | 6.0 mg | 6.0 mg | 6.0 mg | 6.0 mg | 6.0 mg | 6.0 mg |
| Total | | 602.0 mg | 602.0 mg | 602.0 mg | 602.0 mg | 602.0 mg | 602.0 mg |

Details of raw materials used in the Examples and Comparative Examples above are as follows.

- Magnesium oxide: Japanese Pharmacopoeia magnesium oxide (heavy grade) from Kyowa Chemical Industry Co.
- Crystal cellulose: Ceolus PH-101 (Examples 1 to 4 and Comparative Examples 1 to 3) or Ceolus UF-711 (Examples 5 to 8 and Comparative Examples 4 to 6) from Asahi Kasei Corporation

- Croscarmellose sodium: Kiccolate ND-2HS from Nichirin Chemical Industries, Ltd.
- Calcium stearate: Calcium stearate, vegetable, from Taihei Chemical Industrial Co., Ltd.

The physical properties of the magnesium oxide used in these examples were measured, and the results were as follows.

- Average particle size: 7.691 $\mu$m (measured according to laser diffraction scattering using a Microtrac Bell MT3300 EX2)
- Bulk density: 0.249 g/mL (measured with 100 mL stainless steel cup (actual mass value (g)/100 (mL))
- Angle of repose: 41.7° (measured using MT-1 Multitester from Seishin Enterprise Co., Ltd.)

[Production method]

Each raw material was weighed according to the above formulations using an electronic balance (Mettler Toledo, PB5001-S/FACT for weighing 5 kg) on a scale of 1.5 million tablets.

1. Mixing

The weighed raw materials of magnesium oxide and internal additives were put into a polyethylene bag (1100 mm x 600 mm) and mixed by shaking the bag 30 times from side to side. After the mixing, the bulk density was measured using a 100 mL stainless steel cup (actual mass value (g)/100 (mL)).

2. Granulation

The resulting mixture was granulated using a dry granulator (Dry granulator RC-156 from Freund Corporation) under the following granulation conditions to produce granules.

[Table 3]

| | |
|---|---|
| Roll pressure | 8 MPa |
| Roll rotational speed | 12 rpm |
| Screw rotation speed | 26 rpm |
| Degassing | 0 to 10 kPa |
| Oscillator rotation speed | 90 rpm |
| Oscillator wire mesh | 20 mesh (0.8 mm gap) |

After granulation, the following items were evaluated.

- Flake ratio: The masses of a product that had undergone the granulation for one minute (one-minute processed product) (A) and a product by sieving the one-minute processed product with a 1000-$\mu$m sieve (B) were measured, and the flake ratio was calculated from Formula 3 below.
  [Formula 3]

$$\text{Flake ratio (\%)} = \frac{B}{A} \times 100 \qquad \text{Formula 3}$$

- Bulk density: Measured with 100 mL stainless steel cup (actual mass value (g)/100 (mL)
- Particle size distribution: Measured using a particle size distribution analyzer (Laser diffraction scattering particle size analyzer LMS-2000e from Seishin Enterprise Co., Ltd.)

3. External additives

The above granules and the weighed raw materials of external additive were put into a polyethylene bag (1100 mm x 600 mm) and mixed by shaking the bag 30 times from side to side.

After mixing, the following items were evaluated.

- Bulk density: Measured with 100 mL stainless steel cup (actual mass value (g)/100 (mL)
- Angle of repose: Measured using MT-1 Multitester from Seishin Enterprise Co., Ltd.

4. Tableting

The mixed granules and the external additive were tableted using a tableting machine (VIRG, a small high-speed rotary tablet press from Kikusui Seisakusho Ltd.).

[Table 4]

| | |
|---|---|
| Punch | Diameter: 10.5, Two-tiered R, No.29, Cup: 0.95mm |
| | Intrusion angle: 30 degrees, R1: 16.8, R2: 4.5 |
| Die | Diameter: 10.5, All carbide |
| Number of punches | 3 |
| Rotation speed of axis | 60 rpm |
| Tablet mass | 602 mg |
| Tableting pressure | 15.0 kN |
| Tablet thickness | Not determined, since tableting pressure was fixed |

After tableting, the following items were evaluated.

- Weight: (Number of tablets tested: 10)
- Thickness: (Number of tablets tested: 5) Measured using a Thickness Gauge from Peacock Co.
- Hardness: (Number of tablets tested: 10) Measured using DC-50 load cell type tabletop hardness tester from Okada Seiko Co., Ltd.
- Abrasion test: Conducted at 100 rpm (Number of tablets tested: as close as possible to 6.5 g) using Tablet Abrasion Tester TFT-1200 from Toyama Sangyo Co., Ltd.
- Disintegration time: (Number of tablets tested: 6) Measured in accordance with the Japanese Pharmacopoeia, Seventeenth Edition, General Information, Disintegration Test. A disintegration tester (Disintegration Tester NT-20HS from Toyama Sangyo Co., Ltd.) was used to measure the disintegration time of the test tablets in water.
- Tableting condition: Tableting pressure/occurrence of compaction failures was visually checked.
- Suspended particle diameter $D_{50}$ ($\mu$m): (Number of tablets tested: 1) Measured using LMS-2000e laser diffraction scattering particle size analyzer from Seishin Enterprise Co., Ltd. The suspended particle diameter was measured by suspending the test tablets in water.
- Tube passability 5Fr: A catheter syringe (enteral feeding set syringe DS20mL, catheter yellow, from Nipro Corporation) was used. The pusher was removed, one tablet was put in the outer tube, and the pusher was restored. 20mL of warm water at 55°C was sucked into the syringe, the tube tip was covered, and the syringe was let stand for 5 minutes. 5 minutes later, the catheter syringe was rolled over by 90 degrees 15 times by hand, and a feeding tube (Atom Feeding Catheter T; 5 Fr thick, 120 cm long, from Atom Medical Corp.) was connected to the syringe. Another 20 mL of internal suspension and ion-exchanged water for washing was injected to confirm whether the tube was occluded or not. This test was performed three times, and the tube was rated as suitable if it was not occluded, and as unsuitable if it was occluded.
- Compression ratio in the tableting step: Calculated using Formula 4 below, with the filling depth (a) and the compression thickness (b).

Formula 4]

$$\text{Compression ratio (\%)} = \frac{(a-b)}{A} \times 100 \qquad \text{Formula 4}$$

[Results]

The following table shows the results of bulk density measurements of the products after mixing (mixing products).

[Table 5]

| Lot No. | Comparative Example 1 | Comparative Example 2 | Example 1 | Example 2 | Example 3 | Comparative Example 3 |
|---|---|---|---|---|---|---|
| Bulk density of mixed product (g/mL) | 0.241 | 0.228 | 0.240 | 0.233 | 0.227 | 0.226 |

Table 6]

| Lot No. | Comparative Example 4 | Comparative Example 5 | Example 4 | Example 5 | Example 6 | Comparative Example 6 |
|---|---|---|---|---|---|---|
| Bulk density of mixed product (g/mL) | 0.227 | 0.234 | 0.229 | 0.226 | 0.232 | 0.231 |

The bulk density of the mixed product was within the range of from 0.20 to 0.25 (g/mL) for each of the Examples and Comparative Examples, and no significant differences were observed.
The following table shows the results of each evaluation item of the granules after granulation (granulation products).

[Table 7]

| Lot No. | Comparative Example 1 | Comparative Example 2 | Example 1 | Example 2 | Example 3 | Comparative Example 3 |
|---|---|---|---|---|---|---|
| Flake ratio (%) | 95.4 | 95.2 | 94.4 | 93.9 | 93.4 | 93.5 |
| Bulk density of granulated product (g/mL) | 0.627 | 0.618 | 0.602 | 0.596 | 0.555 | 0.545 |
| Processing capability (kg/h) | 30.60 | 30.52 | 29.84 | 27.52 | 26.03 | 23.96 |

[Table 8]

| Lot No. | Comparative Example 4 | Comparative Example 5 | Example 4 | Example 5 | Example 6 | Comparative Example 6 |
|---|---|---|---|---|---|---|
| Flake ratio (%) | 95.2 | 95.2 | 95.1 | 95.0 | 94.2 | 93.2 |
| Bulk density of granulated product (g/mL) | 0.640 | 0.629 | 0.618 | 0.606 | 0.589 | 0.561 |
| Processing capability (kg/h) | 30.84 | 30.38 | 29.68 | 28.84 | 27.48 | 25.20 |

With regard to the granulation ability, the more crystalline cellulose contained as an internal additive, the better the processing capacity and the flake ratio, and the higher the bulk density of the granulated product. It is therefore deemed preferable to include a certain amount of crystalline cellulose as an internal additive.
The following table shows the results of each evaluation item after the addition of the external additives.

[Table 9]

| Lot No. | Comparative Example 1 | Comparative Example 2 | Example 1 | Example 2 | Example 3 | Comparative Example 3 |
|---|---|---|---|---|---|---|
| Bulk density (g/mL) | 0.642 | 0.631 | 0.627 | 0.612 | 0.585 | 0.560 |
| Particle diameter $D_{50}$ (g/mL) | 382.0 | 367.2 | 374.5 | 356.2 | 361.7 | 361.7 |
| Particle diameter 500$\mu$m On (%) | 33.9 | 33.4 | 32.5 | 30.5 | 31.4 | 31.9 |
| Particle diameter 150$\mu$m Pass (%) | 24.9 | 27.2 | 24.2 | 27.7 | 28.4 | 29.3 |

[Table 10]

| Lot No. | Comparative Example 4 | Comparative Example 5 | Example 4 | Example 5 | Example 6 | Comparative Example 6 |
|---|---|---|---|---|---|---|
| Bulk density (g/mL) | 0.677 | 0.657 | 0.652 | 0.623 | 0.597 | 0.574 |
| Particle diameter $D_{50}$ (g/mL) | 396.0 | 376.5 | 376.7 | 371.9 | 341.3 | 373.8 |
| Particle diameter 500$\mu$m On (%) | 36.5 | 35.0 | 34.0 | 33.2 | 30.6 | 34.6 |
| Particle diameter 150$\mu$m Pass (%) | 24.1 | 27.2 | 26.1 | 26.9 | 30.8 | 28.4 |

Comparing the particle diameters after the addition of external additives of Comparative Examples 1 and 4, which do not contain crystalline cellulose as an external additive, and Comparative Examples 3 and 6, which contain 100% crystalline cellulose as an external additive, there was an increase of 150 $\mu$m Pass in Comparative Examples 3 and 6, but there was not much difference in the angles of repose, which were within the range of from 40° to 44°. Thus, no difference in liquidity is expected.

The following table shows the results of each evaluation item after tableting.

[Table 11]

| Lot No. | Comparative Example 1 | Comparative Example 2 | Example 1 | Example 2 | Example 3 | Comparative Example 3 |
|---|---|---|---|---|---|---|
| Filling depth (mm) | 9.87 | 9.95 | 10.67 | 11.44 | 11.81 | 12.95 |
| Compression ratio (%) | 75.88 | 75.78 | 77.13 | 78.58 | 79.25 | 80.93 |
| Hardness (N) | 111 | 118 | 138 | 162 | 172 | 181 |
| Friability (%) 100 rotations | 0.44 | 0.41 | 0.29 | 0.18 | 0.09 | 0.04 |
| Incidence of capping (%) in 100 tablets | 0 | 0 | 0 | 3 | 6 | 12 |
| Disintegration time (seconds) | 10 | 10 | 11 | 11 | 11 | 11 |

(continued)

| Lot No. | Comparative Example 1 | Comparative Example 2 | Example 1 | Example 2 | Example 3 | Comparative Example 3 |
|---|---|---|---|---|---|---|
| Suspended particle diameter $D_{50}$ ($\mu$m) | 74.1 | 83.2 | 90.9 | 97.8 | 96.7 | 113.1 |
| Tableting fault | None | None | None | None | None | None |
| Tube passing ability 5Fr | Good (3/3) | Good (3/3) | Good (3/3) | Good (3/3) | Good (3/3) | Not good (1/3) |

[Table 12]

| Lot No. | Comparative Example 4 | Comparative Example 5 | Example 4 | Example 5 | Example 6 | Comparative Example 6 |
|---|---|---|---|---|---|---|
| Filling depth (mm) | 9.33 | 9.67 | 9.77 | 10.24 | 10.63 | 11.37 |
| Compression ratio (%) | 74.17 | 74.97 | 75.13 | 76.17 | 77.05 | 78.45 |
| Hardness (N) | 91 | 95 | 104 | 109 | 118 | 136 |
| Friability (%) 100 rotations | 0.39 | 0.35 | 0.27 | 0.16 | 0.10 | 0.01 |
| Incidence of capping (%) in 100 tablets | 0 | 0 | 0 | 1 | 3 | 8 |
| Disintegration time (seconds) | 10 | 10 | 11 | 10 | 11 | 11 |
| Suspended particle diameter $D_{50}$ ($\mu$m) | 67.8 | 72.5 | 75.4 | 83.2 | 87.9 | 102.2 |
| Tableting fault | None | None | None | None | None | None |
| Tube passing ability 5Fr | Good (3/3) | Good (3/3) | Good (3/3) | Good (3/3) | Good (3/3) | Good (3/3) |

As the mass ratio of the crystalline cellulose contained as an external additive to that of the crystalline cellulose contained as an internal additive increased (in the order of Comparative Example 1 to Comparative Examples 3 and 4 to Comparative Example 6), the hardness increased and the friability decreased, but the capping rate increased, and agglomeration of suspended particles was observed. It is deemed that as the mass ratio of the crystalline cellulose contained as an external additive to that of the crystalline cellulose contained as an internal additive increased, the ratio of uncompacted crystalline cellulose that can contribute to tablet forming increased to thereby increase the compression ratio during tableting, leading to increased hardness and decreased friability. On the other hand, it is also deemed that insufficient compaction during granulation made it easier for the fine particles that have not fully formed into flakes to hold air, while the bulk density of the entire granules to be tableted also decreased due to uncompacted crystalline cellulose added afterward, whereby insufficient degassing occurred during compression. to cause the granules to hold air. These are thought to be the cause of the increase in the incidence of capping. According to the tube passability test, some clogs were observed for Comparative Example 3, where all of the crystalline cellulose was contained as an external additive. Therefore, when the mass of cellulose and/or cellulose derivative in the total tablet is set at 100, the mass ratio of crystalline cellulose contained as an external additive to reduce friability may be approximately 25 or more, and approximately 90 or less.

The tablet thickness after tableting was within the range of 5.3 to 5.5 mm in all examples, and no significant differences were observed. The disintegration time was also within an appropriate range in all examples.

The above results show that magnesium oxide tablets with both reduced friability and reduced capping incidence can be efficiently produced by adjusting the mass ratio between [the cellulose and/or the cellulose derivative contained as an internal additive] : [the cellulose and/or the cellulose derivative contained as an external additive] to within the range of from 75:25 to 10:90, preferably within the range of from 75:25 to 20:80, more preferably within the range of from 75:25 to 25:75. These ratios also make it possible to achieve both reduction of friability and capping incidence while ensuring the physical properties required for tablet manufacturing, such as formability, granulation, tableting, flowability, and tube passing ability, as well as the physical properties required for magnesium oxide tablets, such as disintegration ability.

INDUSTRIAL APPLICABILITY

The present invention has extremely high applicability for use in the industrial fields which require tablets containing magnesium oxide particles as the main ingredient with both reduced friability and reduced capping incidence, such as the field of pharmaceutical manufacturing and distribution.

**Claims**

1.  A tablet comprising magnesium oxide as an active ingredient, wherein the tablet comprising:

    granules containing magnesium oxide particles and an internal additive; and
    an external additive,
    wherein each of the internal additive and the external additive at least contains a cellulose and/or a cellulose derivative,
    wherein the mass ratio between [the cellulose and/or the cellulose derivative contained as an internal additive] : [the cellulose and/or the cellulose derivative contained as an external additive] is within the range of from 75:25 to 10:90.

2.  The tablet according to claim 1, wherein the cellulose and/or the cellulose derivative is at least one selected from the group consisting of crystalline cellulose, microcrystalline cellulose, powder cellulose, methyl cellulose, ethyl cellulose, carboxyl methyl cellulose, carboxyl methyl cellulose calcium, carboxyl methyl cellulose sodium, hydroxy ethyl cellulose, hydroxy propyl cellulose, hydroxy propyl methyl cellulose, and low substituted hydroxy propyl cellulose.

3.  The tablet according to claim 1 or 2, wherein the content ratio of magnesium oxide particles in the total tablet is 80 to 90 mass %.

4.  The tablet according to any one of claims 1 to 3, wherein the granules contain at least one selected from the group consisting of croscarmellose sodium, corn starch, carmellose calcium, crospovidone and carboxyl starch sodium at an amount of from 1 to 3.5 mass % of the total tablet.

5.  A method for producing a tablet containing magnesium oxide as an active ingredient tablet, comprising:

    mixing magnesium oxide particles with an internal additive including a cellulose and/or a cellulose derivative to prepare a mixture;
    granulating the mixture to prepare granules; and
    adding an external additive including a cellulose and/or a cellulose derivative to the granules and tableting the granules;
    wherein the mass ratio between [the cellulose and/or the cellulose derivative contained as an internal additive] : [the cellulose and/or the cellulose derivative contained as an external additive] is within the range of from 75:25 to 10:90.

6.  The method according to claim 5, wherein the cellulose and/or the cellulose derivative is at least one selected from the group consisting of crystalline cellulose, microcrystalline cellulose, powder cellulose, methyl cellulose, ethyl cellulose, carboxyl methyl cellulose, carboxyl methyl cellulose calcium, carboxyl methyl cellulose sodium, hydroxy ethyl cellulose, hydroxy propyl cellulose, hydroxy propyl methyl cellulose and low substituted hydroxy propyl cellulose.

7.  A tablet produced by a method according to claim 5 or 6.

<table>
<tr><td colspan="2" align="center"><b>INTERNATIONAL SEARCH REPORT</b></td><td>International application No.<br><b>PCT/JP2022/030484</b></td></tr>
</table>

**A.   CLASSIFICATION OF SUBJECT MATTER**

*A61K 33/08*(2006.01)i; *A61K 9/20*(2006.01)i; *A61K 47/32*(2006.01)i; *A61K 47/36*(2006.01)i; *A61K 47/38*(2006.01)i; *A61P 1/04*(2006.01)i; *A61P 1/10*(2006.01)i; *A61P 3/02*(2006.01)i
FI:   A61K33/08; A61K9/20; A61K47/32; A61K47/36; A61K47/38; A61P1/04; A61P1/10; A61P3/02

According to International Patent Classification (IPC) or to both national classification and IPC

**B.   FIELDS SEARCHED**

Minimum documentation searched (classification system followed by classification symbols)

A61K33/08; A61K9/20; A61K47/32; A61K47/36; A61K47/38; A61P1/04; A61P1/10; A61P3/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C.   DOCUMENTS CONSIDERED TO BE RELEVANT**

| Category* | Citation of document, with indication, where appropriate, of the relevant passages | Relevant to claim No. |
|---|---|---|
| X | JP 2015-48316 A (ASAHI FOOD & HEALTHCARE LTD) 16 March 2015 (2015-03-16) claim 1, paragraphs [0008], [0104]-[0114], production example 2 | 1-7 |
| Y | | 1-7 |
| Y | JP 2003-146889 A (KYOWA CHEM IND CO LTD) 21 May 2003 (2003-05-21) claims 1-8, paragraph [0009] | 1-7 |
| Y | JP 2010-517936 A (TAKEDA PHARMACEUTICAL COMPANY LIMITED) 27 May 2010 (2010-05-27) paragraphs [0001]-[0004], example 1 | 1-7 |
| Y | JP 2010-106014 A (TAISHO PHARMACEUTICAL CO LTD) 13 May 2010 (2010-05-13) paragraphs [0001]-[0010], [0022] | 1-7 |

☐ Further documents are listed in the continuation of Box C.    ☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 September 2022** | **04 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2022/030484**

| Patent document cited in search report | | | Publication date (day/month/year) | Patent family member(s) | Publication date (day/month/year) |
|---|---|---|---|---|---|
| JP | 2015-48316 | A | 16 March 2015 | (Family: none) | |
| JP | 2003-146889 | A | 21 May 2003 | EP 1421944 A1 claims 1-8, paragraph [0009] | |
| JP | 2010-517936 | A | 27 May 2010 | US 2009/0318482 A1 paragraphs [0001]-[0006] | |
| JP | 2010-106014 | A | 13 May 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003146889 A

- JP WO2011030659 A

**Non-patent literature cited in the description**

- Japanese Pharmacopoeia. General Information, Tablet Friability Test
- Japanese Pharmacopoeia. General Information, Disintegration Test

- Dietary Reference Intakes: Calcium, Phosphorus, Magnesium, Vitamin D and Fluoride. Institute of Medicine (IOM). Food and Nutrition Board. Academy Press, 1997